# EUROPEAN PATENT APPLICATION

(11) **EP 4 696 520 A1**
(43) Date of publication of application: **18.02.2026**
(21) Application number: 24788671.6
(22) Date of filing: 05.04.2024
(51) Int. Cl.: B43K 19/14, A45D 40/20, A61K 8/00, A61K 8/19, A61K 8/31, A61K 8/37, A61K 8/73, A61K 8/92, A61K 8/98, A61Q 1/10, B43K 19/02

(54) **SHAFT BODY FOR PENCILS OR COSMETICS**

(30) Priority: 14.04.2023 JP 2023066409
(71) Applicant: MITSUBISHI PENCIL COMPANY, LIMITED, Tokyo 140-8537 (JP)
(72) Inventor: KUBO Ryota, Fujioka-shi, Gunma 375-8501 (JP)
(74) Representative: Vossius & Partner Patentanwälte Rechtsanwälte mbB
(86) International application number: PCT/JP2024/014086
(87) International publication number: WO 2024/214647

(57) **Abstract**

[Problem] Provided is a shaft body for a pencil lead (including a colored pencil lead) or a shaft body for a cosmetic such as a rod-shaped cosmetic including an eye liner, an eyebrow pencil, or an eye shadow, which has the same ease of cutting, drop resistance, and water resistance as a conventional wood shaft, has a low impact on the environment, and can have an appearance, including a cut cross-section, in desired color without painting. [Solution] The shaft body for a pencil or a cosmetic according to the present disclosure is a shaft body for a pencil or a cosmetic made by coating a pencil lead or a rod-shaped cosmetic with a shaft body material composition, in which the shaft body material composition contains at least a naturally-derived extender, a naturally-derived binder, and a pigment.

## Description

### Technical Field

The present specification relates to a shaft body for a pencil lead or a cosmetic that has the same ease of cutting, drop resistance, and water resistance as a conventional wood shaft, has a low impact on the environment, and can have an appearance, including a cut cross-section, in desired color without painting.

### Background Art

For a shaft body for a pencil, a rod-shaped cosmetic, or the like, natural wood materials that provide a pleasing tactile experience to the user, such as natural wood materials including red cedar produced in the United States in particular, have been used in the related art because those are soft and easy to cut with a knife or a pencil sharpener. Meanwhile, in recent years, natural wood materials have been decreasing due to excessive logging such as deforestation, and under such circumstances, attention has been paid to the issue of environmental destruction. Red cedar and the like also have become difficult to obtain, and alternative materials have been developed.
In addition, in a case of coloring the shaft body using the natural wood, a method for coloring the shaft body by impregnating the natural wood itself with a liquid containing a dye and a method for applying a coating material to the surface of the shaft body after the shaft body is formed are general. However, in the former, the environmental impact is high due to large energy required during the impregnation and generation of a dye waste liquid; and in the latter, the cross-section at the time of cutting for exposing the pencil lead or the rod-shaped cosmetic remains in its natural wood color.

As a technique for an alternative material of a shaft body for a pencil, a rod-shaped cosmetic, or the like, or a technique of coloring the shaft body, for example, the following techniques 1) to 7) have been known before.
1) A method for producing a plate-shaped material for a pencil shaft, the method including adding caustic soda or a soluble substance having the same effect to old newspaper, pulp wood pieces, sawdust, or other plant fibrous substances, boiling and dissolving the mixture in water to obtain a liquid, molding the liquid into a plate shape, drying the molded product, uniformly permeating one or more of cellulose acetate, stearin, lacquer, urea resin, and the like into the inside of the molded product, and appropriately pressurizing the molded product to obtain a plate shape (see, e.g., Patent Document 1);
2) A pencil including a core material containing a thermoplastic resin, a lubricant, and graphite or a pigment, wherein protruding stripes and recessed stripes are alternately and continuously integrally formed along an axial direction on a rod-shaped outer peripheral surface of the core material; a shaft material containing the thermoplastic resin, cellulose fiber, and a foaming agent formed on an outer side of the core material so as to be in close contact with an uneven surface of the stone material, wherein a part of the cellulose fiber in the shaft material is mixed into an uneven portion of the rod-shaped outer peripheral surface of the core material in a biting manner (see, e.g., Patent Document 2);
3) A pencil in which a core is inserted into a shaft obtained by mixing, with a resin, a cellulose-based fine powder subjected to a grinding treatment, molding the mixture by extrusion or injection molding, and further forming the molded body into a shaft shape of a pencil, in order to provide a pencil that causes no problem even when sharpened with a pencil sharpener and can be produced at low cost because the shaft has excellent processability (see, e.g., Patent Document 3);
4) A method for producing a substantially rod-shaped composite from a core surrounded by at least one polymer obtained by plasticizing the polymer and extruding the polymer onto a continuous core, wherein at least one natural polymer based on lignin is used and a slip agent based on natural oil and/or wax is added to the plasticized polymer before or during extrusion; and further a method for adding natural fibers to a plasticized polymer before or during extrusion; the method for producing writing utensils such as pencils or colored pencils; the method for producing pencils for cosmetic products; a composite coated by applying paint or the like (see, e.g., Patent Document 4);
5) A wood-substituting material for a wood pencil, the wood-substituting material including at least one polymer binder, at least one organic filler, at least one inorganic filler, at least one compatibilizer, at least one wax, at least one coloring pigment, and at least one additive, wherein
   the at least one compatibilizer forms a chemical bond between the at least one polymer binder and the at least one organic filler,
   the ratio of the at least one compatibilizer to the at least one wax is in a range of 1:2 to 1:6, and
   the sum of the organic filler and the inorganic filler is 80 wt.% or less (see, e.g., Patent Document 5);
6) A rigid fiberboard for use as a pencil shaft, the rigid fiberboard including fibers selected from the group consisting of natural fibers, synthetic fibers, and mixtures thereof, a binder, a filler, a lubricant, and a waterproofing agent, wherein
   the natural fibers are cellulose fibers obtained from a source selected from the group consisting of wood pulp, linters, denim fibers, newspaper, cardboard boxes, kraft paper bags, bleached paper, kraft paper, cotton linters, and mixtures thereof,
   the binder is selected from the group consisting of thermosetting resin binders, water-soluble gums, and mixtures thereof,
   the water-soluble gums are selected from the group consisting of guar gum, carboxymethyl cellulose, hydroxyethyl cellulose, and mixtures thereof,
   the filler is an inorganic particulate material selected from the group consisting of perlite, vermiculite, glass microbubbles, and mixtures thereof; The fiberboard further containing at least one dye; A pencil including a marking core, and a shaft enclosing the core, wherein the shaft is formed from a fiberboard containing fibers, a binder, a filler, a lubricant, and a waterproofing agent (see, e.g., Patent Document 6); and
7) For providing a biodegradable pencil type cosmetic synthetic shaft that has excellent physical properties, such as rigidity, cutting property, and oil fat penetration resistance, and a good feeling of use as a synthetic shaft replacing a wooden shaft, that is easily decomposed after disposal, that can be burned with a low calorific value, and that does not generate a harmful substances at the time of incineration, and a production method thereof, a hollow cylindrical biodegradable pencil type cosmetic synthetic shaft (see, e.g., Patent Document 7) and the like are known. The hollow cylindrical biodegradable pencil type cosmetic synthetic shaft is molded using, as a base material, a reinforcing filler and a biodegradable thermoplastic material including a starch ester produced by substituting hydrogen of a reactive hydroxyl group of the same starch molecule with a short chain acyl group and a long chain acyl group.

However, in the above Patent Document 1, the plant fibrous substances need to be boiled and dissolved, and in the above Patent Documents 2 to 5, a thermoplastic or thermosetting resin is used, and therefore, heating is essential at the time of coloring, molding, or curing, and thus, there is a problem in that the impact on the environment is still high. In addition, in the above Patent Documents 6 and 7, natural fibers, a starch ester, and the like are used, but there is a problem in that the impact on the environment is still high, and the aesthetic treatment of an appearance, including a cut cross-section, is still insufficient.

### Citation List

### Patent Document

Patent Document 1: JP S37-2728 B (Claims, etc.)
Patent Document 2: JP S60-204399 A (Claims, etc.)
Patent Document 3: JP H06-255296 A (Claims, etc.)
Patent Document 4: JP 2002-529273 T (Claims, etc.)
Patent Document 5: JP 2011-528046 T (Claims, etc.)
Patent Document 6: JP H05-96899 A (Claims, etc.)
Patent Document 7: JP 2001-192502 A (Claims, etc.)

### Summary of Invention

### Technical Problem

The present disclosure has been made to solve the above-described known problems and the like, and an object of the present disclosure is to provide a shaft body for a pencil lead or a cosmetic that has the same ease of cutting, drop resistance, and water resistance as a conventional wood shaft, has a low impact on the environment, and can have an appearance, including a cut cross-section, in desired color without painting.

### Solution to Problem

As a result of intensive study on the above-described known problems, the present disclosers have found that the shaft body for a pencil or a cosmetic for the above object can be obtained from a shaft body for a pencil or a cosmetic made by coating a pencil lead or a rod-shaped cosmetic with a shaft body material composition, in which the shaft body material composition contains at least an extender having specific physical properties, a binder, and a pigment, and have completed the present disclosure.

That is, the shaft body for a pencil or a cosmetic of the present disclosure is characterized by a shaft body for a pencil or a cosmetic made by coating a pencil lead or a rod-shaped cosmetic with a shaft body material composition, in which the shaft body material composition contains at least a naturally-derived extender, a naturally-derived binder, and a pigment.

The shaft body material composition preferably has a natural origin content (excluding water) of 75% or more.

The pencil according to the present disclosure is a pencil including at least a pencil lead and a shaft body made by coating and molding the pencil lead, in which the shaft body is a shaft body having the above-described configuration. The rod-shaped cosmetic product according to the present disclosure is a rod-shaped cosmetic product including at least a rod-shaped cosmetic and a shaft body made by coating and molding the rod-shaped cosmetic, in which the shaft body is a shaft body having the above-described configuration.

### Advantageous Effects of Invention

According to the present disclosure, it is possible to provide a shaft body for a pencil lead or a cosmetic that has the same ease of cutting, drop resistance, and water resistance as a known wood shaft, has a low impact on the environment, and can have an appearance, including a cut cross-section, in desired color without painting, as well as a pencil, a rod-shaped cosmetic product, or the like including the foregoing shaft body.
The object and effects of the present disclosure can be recognized and obtained especially using the components and combinations indicated in the claims. Both general explanation described above and detailed explanation described below are exemplary and explanatory and do not limit the present disclosure described in the claims.

### Description of Embodiments

Hereinafter, embodiments of the present disclosure will be described in detail. Note that the technical scope of the present disclosure is not limited to the embodiments described below but includes the invention described in the claims and equivalents thereof. In addition, the present disclosure can be implemented based on the contents disclosed in the present specification and technical common knowledge (including design matters and obvious matters) in the art.

The shaft body for a pencil or a cosmetic of the present disclosure is a shaft body for a pencil or a cosmetic made by coating a pencil lead or a rod-shaped cosmetic with a shaft body material composition, in which the shaft body material composition contains at least a naturally-derived extender, a naturally-derived binder, and a pigment.

Examples of the naturally-derived extender to be used in the present disclosure include sawdust, wood flour, powdery cellulose, and naturally-derived inorganic powder obtained from wood materials of natural origin.
The sawdust that can be used may be derived from hardwood or softwood and is not particularly limited. Specific examples of tree species of the sawdust to be used in the present disclosure include cedar, pine, larch, red pine, Sakhalin Fir, cypress, beech, horse chestnut, Quercus serrata, Quercus crispula, oak, Japanese linden, birch, Japanese Elm, lauan, and Western hemlock. In particular, cedar, cypress sapwood, beech, horse chestnut, Quercus crispula, or the like is preferably used. In addition, waste materials generated during production of these wood materials may be effectively utilized. Further, at least one kind (one kind or a combination of two or more kinds; the same applies hereinafter) of these kinds of sawdust can be used.
Regarding the size of the sawdust, from the viewpoint of dispersibility, moldability, and the like, it is desirable that the average particle size is 10 mesh or more, and it is further desirable to use sawdust in a range of 200 mesh or less from the viewpoint of an energy cost required for pulverization.

As the wood flour that can be used, for example, at least one kind of wood flour obtained from each tree species exemplified in the above-described sawdust, wood flour obtained from the above-described sawdust or waste wood, sander powder, or the like can be used. To further improve the dispersibility and moldability, the upper limit of the average particle size of the wood flour is preferably, for example, 2000 µm or less, and the lower limit thereof is preferably, for example, 100 µm or more. However, the wood flour may be in the form of a microfibrillated fiber. Note that the average particle size in the present disclosure can be a value measured in accordance with a laser diffraction/scattering method.

The powdery cellulose that can be used is preferably cellulose powder obtained by finely pulverizing naturally-derived cellulose fibers of a plant, and the average particle size thereof is 100 µm or less, and preferably in a range of 10 to 50 µm. As the powdery cellulose, for example, crystalline cellulose powder having a certain particle size distribution produced by a method for purifying, drying, and pulverizing/sieving an undecomposed residue obtained after acid hydrolysis of selected pulp may be used, or a commercially available product such as the trade name "KC Flock" (available from Nippon Paper Chemicals Co., Ltd.), the trade name "Ceolus" (available from Asahi Kasei Chemicals Corporation), or the trade name "Avicel" (available from FMC Corporation) may be used.

The naturally-derived inorganic powder that can be used includes at least one of: talc, calcium carbonate, huntite, kaolin, sericite, bentonite, titanium oxide, or the like.
The upper limit of the average particle size of these kinds of inorganic powder is preferably, for example, 50 µm or less, and the lower limit thereof is preferably, for example, 1 µm.

The content of these naturally-derived extenders is preferably 60 to 90 mass%, and more preferably 70 to 80 mass%, relative to the total amount of the shaft body material composition. When the content of the naturally-derived extender is 60 mass% or more, usable cutting property can be achieved, and when the content is 90 mass% or less, usable strength can be achieved.
In addition, when the proportion of the inorganic powder in the naturally-derived extender is 60 mass% or less, drop resistance of the shaft body is improved, and thus in a case where the shaft body is used for a pencil or a cosmetic, it is possible to prevent breakage inside the pencil lead or the rod-shaped cosmetic.

As the naturally-derived binder to be used in the present disclosure, at least one of cellulose fibers (including nanofibers), methylcellulose or a salt thereof, carboxymethylcellulose or a salt thereof, hydroxymethylpropylcellulose or a salt thereof, cellulose acetate or a salt thereof, sodium alginate, or the like can be used. Examples of the salt of each kind of cellulose described above include sodium, ammonium, and calcium.
These binders are preferably water-soluble, and further, the naturally-derived binder is preferably carboxymethyl cellulose ammonium from the viewpoint of imparting water resistance.

The content of these naturally-derived binders is preferably 10 to 40 mass%, and more preferably 20 to 30 mass%, relative to the total amount of the shaft body material composition.
When the content of the naturally-derived binder is 10 mass% or more, usable strength can be achieved, and when the content is 40 mass% or less, usable cutting property can be achieved.

The shaft body material composition according to the present disclosure may further contain, in addition to the naturally-derived extender and the naturally-derived binder, a crosslinking agent for crosslinking the binder, from the viewpoint of further exhibiting the effects of the present disclosure and imparting water resistance.
The crosslinking agent for crosslinking the binder is preferably a crosslinking agent having a carboxylic acid group that ester-crosslinks the binder, and specific examples thereof include succinic acid, malic acid, maleic acid, tartaric acid, citric acid, and polyacrylic acid. The content of the crosslinking agent is preferably 0.1 to 5 mass%, and more preferably 0.5 to 2 mass%, relative to the total amount of the shaft body material composition, from the viewpoint of exhibiting water resistance, suppressing a decrease in strength due to moisture absorption, and suppressing discoloration of the material.

Examples of the pigment used in the present disclosure include inorganic pigments and organic pigments. In the present disclosure, a shaft body for a pencil or a cosmetic material having a low impact on the environment is formed using the above-described naturally-derived extender, naturally-derived binder, and the like, and thus it is preferable to use a pigment having a low impact on the environment as the pigment. Therefore, it is not preferable to use a resin particle pigment containing a pigment, a white plastic pigment, a hollow resin particle, a thermochromic pigment, a photochromic particle, or the like, or a composite particle thereof.

Examples of the inorganic pigment that can be used include at least one of carbon black, titanium black, zinc oxide, colcothar, aluminum, chromium oxide, iron black, cobalt blue, iron oxide yellow, viridian, zinc sulfide, lithopone, cadmium yellow, vermilion, cadmium red, chrome yellow, molybdate orange, zinc chromate, strontium chromate, white carbon, clay, talc, ultramarine blue, precipitated barium sulfate, barite powder, calcium carbonate, white lead, navy blue and white, Prussian blue, manganese violet, aluminum powder, brass powder, titanium oxide, zinc oxide, silica powder, or lithopone.

Examples of the organic pigment that can be used include azo lake, insoluble azo pigments, chelate azo pigments, phthalocyanine pigments, perylene or perinone pigments, and nitroso pigments. More specific examples include at least one of C.I. Pigment Blue 15, C.I. Pigment Blue 17, C.I. Pigment Blue 27, C.I. Pigment Red 5, C.I. Pigment Red 22, C. I. Pigment Red 38, C. I. Pigment Red 48, C.I. Pigment Red 49, C.I. Pigment Red 53, C. I. Pigment Red 57, C. I. Pigment Red 81, C. I. Pigment Red 104, C.I. Pigment Red 146, C.I. Pigment Red 245, C.I. Pigment Yellow 1, C.I. Pigment Yellow 3, C. I. Pigment Yellow 12, C. I. Pigment Yellow 13, C. I. Pigment Yellow 14, C. I. Pigment Yellow 17, C.I. Pigment Yellow 34, C. I. Pigment Yellow 55, C. I. Pigment Yellow 74, C.I. Pigment Yellow 95, C. I. Pigment Yellow 166, C. I. Pigment Yellow 167, C. I. Pigment Orange 5, C. I. Pigment Orange 13, C. I. Pigment Orange 16, C.I. Pigment Violet 1, C.I. Pigment Violet 3, C.I. Pigment Violet 19, C.I. Pigment Violet 23, C.I. Pigment Violet 50, or C.I. Pigment Green 7.
Among these pigments, inorganic pigments, particularly iron black, iron oxide yellow, colcothar, titanium oxide, and the like are desirably used from the viewpoint of light resistance and heat resistance. In addition, the average particle size of these pigments is desirably 0.1 to 2 µm from the viewpoint of preventing a decrease in strength and exhibiting sufficient coloring power.
In the present disclosure (including Examples, etc.), "average particle size" is a value of D50 measured with a particle size analyzer [MICROTRAC HRA9320-X100 (available from Nikkiso Co., Ltd.)].

The content of these pigments is preferably 0.2 to 5 mass%, and more preferably 0.5 to 2 mass%, relative to the total amount of the shaft body material composition from the viewpoint of not causing a decrease in strength and obtaining good color reproduction.
Furthermore, the shaft body material composition of the present disclosure may appropriately contain an antimicrobial agent, a preservative, a fragrance, and the like as necessary, in addition to the naturally-derived extender, the naturally-derived binder, the crosslinking agent, and the pigment described above.

These shaft body material compositions preferably have a natural origin content (excluding water) of 75% or more from the viewpoint of further exhibiting the effects of the present disclosure and from the viewpoint of an impact on the environment.
The term "natural origin content (excluding water) " refers to an index expression according to ISO 16128, and when the natural origin content (excluding water) is 75% or more, and more preferably 85% or more, a shaft body for a pencil lead or a cosmetic that has an extremely low impact on the environment can be further obtained.
The natural origin content (excluding water) of the shaft body material composition can be set to 75% or more by appropriately selecting the naturally-derived extender, the naturally-derived binder, and the pigment described above.

The shaft body for a pencil or a cosmetic of the present disclosure is obtained as follows. A blended composition including the naturally-derived extender, the naturally-derived binder, the pigment, and the like described above is dry-mixed using a kneading apparatus such as a Henschel mixer, a planetary mixer, a kneader, or the like, and then, water (purified water, distilled water, or the like) is added to the mixture to achieve a moisture content of 40 to 80%, thereby obtaining a slurry. The slurry is further kneaded while being warmed, degassed, or the like to obtain a clay-like mixture having a moisture content of about 30 to 60%. An extrusion-molding machine or the like is used to get the mixture on an outer periphery of a pencil lead or a cosmetic to be coated using a die having a predetermined diameter, and the resulting product is dried, whereby a pencil or a cosmetic is obtained. Alternatively, a pencil or a cosmetic can also be obtained by a method in which the kneaded product is molded into a round bar or a plate shape using an extrusion-molding machine or the like with a die having a predetermined diameter or a T-die and then dried to produce a molded body, to which a pencil lead or a cosmetic is inserted after processed. Note that the physical properties of the shaft body can be controlled in a wide range by a pressing pressure in extrusion molding or the like, a moisture content, and combination of the naturally-derived extender, the naturally-derived binder, and the like, and thus, a shaft body suitable for a pencil or a cosmetic, which can satisfy required texture, strength, moldability, hue of an appearance including a cut cross-section, and the like of the shaft body, can be obtained.

In the present disclosure, a composition, a form, and the like of the pencil lead to be coated with the shaft body material composition are not particularly limited, and examples of the pencil lead include a fired pencil lead (including a fired colored pencil lead) and a non-fired pencil lead (including a non-fired colored pencil lead). Examples of the fired pencil lead include a fired pencil lead obtained by mixing, dispersing, and kneading materials including a coloring component such as graphite, an organic binder such as a vinyl chloride resin, a vinylidene chloride resin, a vinyl acetate resin, chlorinated polyethylene, polyvinyl alcohol, an acryl amide resin, a chlorinated paraffin resin, a phenol resin, a furan resin, an urea resin, or butyl rubber, a plasticizer such as a phthalic acid ester, a solvent such as methyl ethyl ketone or water, a stabilizer such as a stearate, a lubricant such as stearic acid, and a filling material such as carbon black, extrusion-molding the kneaded mixture into a thin line shape, heat-treating the molded product to a firing temperature to obtain a lead body, and impregnating the obtained lead body with an oily substance such as a silicone oil, liquid paraffin, spindle oil, squalane, or an α-olefin oligomer, or a wax.

A composition, a form, and the like of the rod-shaped cosmetic to be coated with the shaft body material composition are not particularly limited, and examples thereof include a cosmetic obtained by kneading blending components to be used for an eye liner, an eyebrow pencil, a lip liner, a concealer pencil, an eye shadow pencil, or the like, for example, a wax such as oil and fat, wax, fatty acid, or hydrocarbon, or a clay as a binder, a colorant, and an extender, and molding the mixture into a rod shape. The rod-shaped cosmetic may be a fired rod-shaped cosmetic or a non-fired rod-shaped cosmetic.

The shaft body for a pencil lead or a cosmetic of the present disclosure configured as described above has the same ease of cutting, drop resistance, and water resistance as a conventional wood shaft, can have an appearance, including a cut cross-section, in desired color, and has a low impact on the environment. A known molding machine or the like used for molding a pencil or a cosmetic can be used without modification, and thus, it is possible to easily produce the shaft body for a pencil lead or a cosmetic.

The pencil according to the present disclosure is characterized in that at least, the shaft body made by coating and molding the above-described pencil lead is the shaft body having the above-described configuration, and the rod-shaped cosmetic product according to the present disclosure is characterized in that at least, the shaft body made by coating and molding the above-described rod-shaped cosmetic is the shaft body having the above-described configuration.
In the present disclosure, the pencil lead or the rod-shaped cosmetic can be molded into any shape, for example, a shape having a cross-sectional shape such as a circular shape (including an elliptical shape) or a polygonal shape such as a triangular shape or a hexagonal shape, or a tubular shape, and thus can be formed into a pencil or a rod-shaped cosmetic product having each shape by various processing methods such as coating molding and cutting processing.

### Examples

Hereinafter, the present disclosure will be described using Examples and Comparative Examples, but the present disclosure is not limited to these Examples.
For the pencils of Examples 1 to 9 and Comparative Examples 1 to 3, pencil leads obtained by the following production method were used. After 60 mass% of graphite and 40 mass% of clay were dry-mixed by a mixing Henschel mixer, purified water was added thereto to achieve a moisture content of 40%, followed by kneading. The obtained slurry was kneaded while being warmed and degassed to obtain a clay-like mixture having a moisture content of about 30%. This mixture was molded using a hydraulic plunger-type extrusion-molding machine with a die having a diameter of 2.5 mm, and the molded body was dried under an environment of 60°C, and then fired at 800 to 1400°C for 12 to 24 hours to obtain a lead body having a diameter of 2.0 mm. The obtained lead body was impregnated with an α-olefin oligomer to obtain a pencil lead having a diameter of 2.0 mm and a hardness of HB.

### (Example 1)

Sawdust of cedar wood material (16 mesh) 69 mass%
Black iron oxide 1 mass%
Carboxymethylcellulose ammonium 30 mass%
The natural origin content (excluding water) of this shaft body material composition was 91.5%.

After the blended composition was dry-mixed by a Henschel mixer, purified water was added thereto to achieve a moisture content of 50%, followed by kneading.

The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture.

The molded body was dried under an environment of 60°C and heated at 180°C to make the binder water-insoluble, thereby obtaining a pencil having a black appearance.

### (Example 2)

Huntite powder (average particle size 2 µm) 89 mass%
Black iron oxide 1 mass%
Cellulose powder (average particle size 45 µm) 10 mass%
The natural origin content (excluding water) of this shaft body material composition was 99.0%.

After the blended composition was dry-mixed by a Henschel mixer, purified water was added thereto to achieve a moisture content of 50%, followed by kneading.

The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture.

The molded body was dried under an environment of 60°C to obtain a pencil having a black appearance.

### (Example 3)

Talc (average particle size 8 µm) 29 mass%
Cellulose powder (average particle size 45 µm) 60 mass%
Black iron oxide 1 mass%
Carboxymethylcellulose ammonium 10 mass%
The natural origin content (excluding water) of this shaft body material composition was 96.5%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C and heated at 180°C to make the binder water-insoluble, thereby obtaining a pencil having a black appearance.

### (Example 4)

Talc (average particle size 8 µm) 28.5 mass%
Cellulose powder (average particle size 45 µm) 60 mass%
C.I. Pigment Red 245 1.5 mass%
Carboxymethylcellulose ammonium 10 mass%
The natural origin content (excluding water) of this shaft body material composition was 97.0%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 50%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C and heated at 180°C to make the binder water-insoluble, thereby obtaining a pencil having a red appearance.

### (Example 5)

Talc (average particle size 8 µm) 28 mass%
Cellulose powder (average particle size 45 µm) 60 mass%
Iron oxide yellow 2 mass%
Carboxymethylcellulose ammonium 10 mass%
The natural origin content (excluding water) of this shaft body material composition was 95.5%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C and heated at 180°C to make the binder water-insoluble, thereby obtaining a pencil having a yellow appearance.

### (Example 6)

Talc (average particle size 8 µm) 28 mass%
Cellulose powder (average particle size 45 µm) 60 mass%
C.I. Pigment Yellow 3 0.5 mass%
C.I. Pigment Red 22 0.4 mass%
Colcothar 1.1 mass%
Carboxymethylcellulose ammonium 10 mass%

The natural origin content (excluding water) of this shaft body material composition was 95.5%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C and heated at 180°C to make the binder water-insoluble, thereby obtaining a pencil having a brown appearance.

### (Example 7)

Talc (average particle size 8 µm) 29 mass%
Cellulose powder (average particle size 45 µm) 60 mass%
Iron oxide black 1 mass%
Sodium carboxymethylcellulose 10 mass%
The natural origin content (excluding water) of this shaft body material composition was 94.1%.

After the blended composition was dry-mixed by a Henschel mixer, a 5% aqueous succinic acid solution was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C, and then heated at 180°C to crosslink the binder, thereby obtaining a pencil having a black appearance.

### (Example 8)

Talc (average particle size 8 µm) 29 mass%
Cellulose powder (average particle size 45 µm) 60 mass%
Iron oxide black 1 mass%
Methylcellulose 10 mass%
The natural origin content (excluding water) of this shaft body material composition was 96.5%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C to obtain a pencil having a black appearance.

### (Example 9)

Talc (average particle size 8 µm) 29 mass%
Cellulose powder (average particle size 45 µm) 60 mass%
Black iron oxide 1 mass%
Methylcellulose 10 mass%
The natural origin content (excluding water) of this shaft body material composition was 94.1%.

After the blended composition was dry-mixed by a Henschel mixer, a 5% aqueous succinic acid solution was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C, and then heated at 180°C to crosslink the binder, thereby obtaining a pencil having a black appearance.

### (Example 10)

### <Preparation of Solid Cosmetic>

Beeswax 20 parts
Ozokerite 10 parts
Microcrystalline wax 10 parts
Carnauba wax 8 parts
Vaseline 7 parts
Lanolin 5 parts
Liquid paraffin 7 parts
Isopropyl myristate 4 parts

The above blending materials were dissolved, 10 parts of iron black and 19 parts of colcothar were added thereto, and the mixture was stirred and dispersed, followed by kneading by a mixer. The kneaded mixture was cooled to room temperature, and then molded by an extrusion-molding machine to obtain a brown solid cosmetic of 2.0 mm.

### (Preparation of Rod-Shaped Cosmetic Product)

Talc (average particle size 8 µm) 29 mass%
Cellulose powder (average particle size 45 µm) 60 mass%
Black iron oxide 1 mass%
Methylcellulose 10 mass%
The natural origin content (excluding water) of the shaft body material composition was 96.5%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat the solid cosmetic having a diameter of 2.0 mm with the mixture. The molded body was dried under an environment of 60°C to obtain a rod-shaped cosmetic product having a black appearance.

### (Comparative Example 1)

Pencil (pencil made by cutting an incense cedar plate material impregnated with 2 wt.% of paraffin wax and covering a pencil lead having a diameter of 2.0 mm and a hardness of HB therewith. The surface of the shaft body is colored using a paint containing iron oxide black.)

### (Comparative Example 2)

This is a pencil in which a known recycled natural material is used and a thermoplastic resin is used as a binder, and thus a non-naturally-derived material is used, and the surface of the shaft body is coated with a thermoplastic resin containing a coloring material. The shaft body has the following composition and has a natural origin content of 70.0%.
Polypropylene (PP) 20 mass%
Wood flour 70 mass%
Maleic anhydride-grafted polypropylene 1 mass%
Amide wax 3 mass%
Stearic acid 1 mass%
Boron nitride 5 mass%

### (Comparative Example 3)

Talc (average particle size 8 µm) 30 mass%
Cellulose powder (average particle size 45 µm) 60 mass% Carboxymethylcellulose ammonium 10 mass%
The natural origin content (excluding water) of this shaft body material composition was 97.5%.

After the blended composition was dry-mixed by a Henschel mixer, purified water was added thereto to achieve a moisture content of 50%, followed by kneading. The obtained slurry was kneaded while being warmed and degassed to obtain a clay-like mixture having a moisture content of about 40%. A hydraulic plunger-type extrusion-molding machine was used with a die having a diameter of 9.0 mm to coat an outer periphery of the pencil lead having a diameter of 2.0 mm and a hardness of HB with the mixture. The molded body was dried under an environment of 60°C and heated at 180°C to make the binder water-insoluble, thereby obtaining a pencil having a white appearance.

For each of the pencils and the rod-shaped cosmetic products of Examples 1 to 10 and Comparative Examples 1 to 3 obtained as described above, the natural origin content was calculated by the following method, and the ease of cutting and the appearance including the cut cross-section were evaluated by the following evaluation method.

The results are shown in Table 1 below.

### (Method for Calculating Natural Origin Content)

The natural origin content (excluding water) of each of the shaft bodies of Examples 1 to 10 and Comparative Examples 1 to 3 is obtained by calculating a composition ratio in percentage using a weight excluding water of a natural raw material and a naturally-derived portion in a naturally-derived raw material, defined in ISO 16128, from weight ratios of raw materials blended in the shaft material excluding a lead. The higher this value is, the higher the proportion of an animal- or plant-derived material as well as the smaller an amount of a petroleum-derived material used is, which can indicate that the impact on the environment is low.

### (Evaluation Method for Ease of Cutting)

For each of the pencils and the rod-shaped cosmetic products of Examples 1 to 10 and Comparative Examples 1 to 3, after the tip was trimmed to have a diameter of 2 mm using a pencil sharpener, the product was cut by using a pencil sharpener mounted on a fixture connected to a rotation moment measuring apparatus and rotating the end opposite to the pencil sharpener by hand until the lead became sharp. A peak value of a rotation moment during this period was measured and evaluated for ease of cutting (cutting property) according to the following evaluation criteria. It can be said that when this value is less than 0.1 N, the pencil is easy to cut, and when the value exceeds 0.2 N·m, the pencil is hard and difficult to cut.

Evaluation criteria:
A: can be easily cut (less than 0.1 N·m)
B: can be cut (0.1 N·m or more and less than 0.2 N·m)
C: hard to be cut (0.2 N·m or more)

### (Evaluation Method for Appearance Including Cut Cross-Section)

For each of the pencils and the rod-shaped cosmetic products of Examples 1 to 10 and Comparative Examples 1 to 3, the cut surface was exposed using a pencil sharpener, the colors of the shaft body and the cut surface were observed, and the appearance including the cut cross-section was evaluated according to the following evaluation criteria.

Evaluation criteria:
A: Both the shaft body and the cut surface are in desired color.
B: The surface of the shaft body is in desired color, but the cut surface is not colored.
C: Not colored and not in any color

**[Table 1]**

| | Natural origin content | Ease of cutting | Appearance including cut cross-section |
|---|---|---|---|
| Example 1 | 91. 50% | B | A |
| Example 2 | 99.00% | B | A |
| Example 3 | 96.50% | A | A |
| Example 4 | 97.00% | A | A |
| Example 5 | 95.50% | A | A |
| Example 6 | 95.50% | A | A |
| Example 7 | 94.10% | A | A |
| Example 8 | 96.50% | A | A |
| Example 9 | 94.10% | A | A |
| Example 10 | 96.50% | A | A |
| Comparative Example 1 | 97.90% | A | B |
| Comparative Example 2 | 70.00% | B | B |
| Comparative Example 3 | 97.50% | A | C |

In consideration of the evaluation results in Table 1 above, it has been confirmed that Examples 1 to 10 within the scope of the present disclosure have a low impact on the environment and can easily have an appearance including a cut cross-section, in desired color while having the same ease of cutting as the known Comparative Examples 1 to 3. In addition, in each of Examples 3 to 10, free falling was performed from a height of 75 cm to a concrete floor ten times for evaluation by setting the inorganic powder ratio in the extender to 60% or less while maintaining a high natural origin content. As a result, it has been confirmed that the pencil or the cosmetic main body, and the pencil lead or the rod-shaped cosmetic were not damaged and had sufficient drop resistance. Furthermore, for each of the pencils in which the naturally-derived binder is made water-insoluble by using carboxymethylcellulose ammonium in Examples 1 and 3 to 7, or is crosslinked by a crosslinking agent having a carboxylic acid group that performs ester crosslinking in Example 9, water resistance (water resistance after immersion of the produced pencil in purified water for 24 hours) was evaluated. As a result, it has been confirmed that the pencil was not broken down and it was possible to exhibit sufficient water resistance.

### Industrial Applicability

It is possible to obtain a shaft body for a pencil or a cosmetic suitable for pencil leads (including colored pencil leads) and rod-shaped cosmetics such as an eye liner, an eyebrow pencil, and an eye shadow.

## Claims

1. A shaft body for a pencil or a cosmetic comprising a pencil lead or a rod-shaped cosmetic coated with a shaft body material composition, wherein the shaft body material composition comprises at least a naturally-derived extender, a naturally-derived binder, and a pigment.

2. The shaft body for a pencil or a cosmetic according to claim 1, wherein the shaft body material composition has a natural origin content (excluding water) of 75% or more.

3. A pencil comprising at least a pencil lead and a shaft body coating and molding the pencil lead, wherein the shaft body is the shaft body described in claim 1 or 2.

4. A rod-shaped cosmetic product comprising at least a rod-shaped cosmetic and a shaft body coating and molding the rod-shaped cosmetic, wherein the shaft body is the shaft body described in claim 1 or 2.
